# EUROPÄISCHE PATENTANMELDUNG

(11) **EP 4 830 696 A1**
(43) Veröffentlichungstag der Anmeldung: **28.10.2026**
(21) Anmeldenummer: 26165993.2
(22) Anmeldetag: 19.03.2026
(51) Int. Cl.: A61K 9/00, A61K 9/08, A61K 31/00

(54) **SYSTEM ZUR BEREITSTELLUNG GEBRAUCHSFERTIGER ACETYLCHOLIN-INJEKTIONSLÖSUNGEN**

(30) Priorität: 23.04.2025 DE 102025115542
(71) Anmelder: Robert Bosch Gesellschaft für medizinische Forschung mbH, 70376 Stuttgart (DE)
(72) Erfinder: ONG, Peter., Stuttgart 70195 (DE); BEINSTEINER, Thomas Muerdter., Weinstadt 71384 (DE)
(74) Vertreter: Bee, Joachim

(57) **Zusammenfassung**

Die vorliegende Erfindung betrifft ein System zur Bereitstellung gebrauchsfertiger Acetylcholin-Injektionslösungen mit unterschiedlichen Acetylcholin-Konzentrationen. Das System weist mindestens fünf erste Aufnahmeeinheiten auf, wobei jede der fünf ersten Aufnahmeeinheiten eine Menge an Acetylcholin oder an einem Acetylcholin-Analogon als Feststoff enthält, wobei die enthaltenen Mengen (A; B; C; D) an Acetylcholin oder an dem Acetylcholin-Analogon zwischen den fünf ersten Aufnahmeeinheiten unterschiedlich sind, derart, dass die Menge an Acetylcholin oder an dem Acetylcholin-Analogon in vier der mindestens fünf ersten Aufnahmeeinheiten jeder nachfolgenden ersten Aufnahmeeinheit größer ist als in der unmittelbar vorhergehenden ersten Aufnahmeeinheit, und die Menge an Acetylcholin oder an dem Acetylcholin-Analogon in der fünften der mindestens fünf ersten Aufnahmeeinheit kleiner ist als die höchste Menge (D) in den vier ersten Aufnahmeeinheiten. Das System zielt ab auf eine Anwendung im Bereich der kardiovaskulären Medizin, genauer gesagt dem intrakoronaren Acetylcholin-Provokationstest zur Erkennung einer CVD während einer Herzkatheteruntersuchung.

## Beschreibung

Die vorliegende Erfindung betrifft ein System zur Bereitstellung gebrauchsfertiger Acetylcholin-Injektionslösungen, die Verwendung des Systems in der Diagnostik, sowie Verfahren zur Herstellung gebrauchsfertiger Acetylcholin-Injektionslösungen, wobei das System bereitgestellt wird. Solche Systeme, die Verwendung und Verfahren sind im Bereich der kardiovaskulären Diagnostik anwendbar und insbesondere für den intrakoronaren Acetylcholin-Provokationstest.

### Stand der Technik

Koronare vasomotorische Störungen (Coronary Vasomotor Disorder; CVD) spielen eine wesentliche Rolle in der Pathogenese von Angina Pectoris und koronarer Herzkrankheit. Diese Erkrankungen sind dadurch gekennzeichnet, dass der Tonus der Gefäßmuskulatur der Koronargefäße nicht korrekt reguliert wird, um die Blut- und Sauerstoffversorgung des Herzens entsprechend der Anforderungen anzupassen. Ein zentraler Faktor in der Steuerung dieser Prozesse ist Acetylcholin (ACh), ein bedeutender Neurotransmitter, der auch für die Regulation des koronaren Gefäßtonus verantwortlich ist.

Zur Diagnose von CVD kann ein intrakoronarer Provokationstest mit der Applikation von ACh durchgeführt werden. Wenn durch die Gabe von ACh ein Vasospasmus der Koronargefäße ausgelöst wird, gilt dies als Indiz für das Vorliegen einer CVD. Der Test ermöglicht eine funktionelle Beurteilung der koronaren Endothelfunktion und glatten Gefäßmuskulatur und dient als wichtiges Werkzeug zur Identifikation von Patienten mit vasospastischer Angina oder koronarer Mikrozirkulationsstörung.

Zur Durchführung des intrakoronaren ACh-Provokationstests werden beginnend mit beispielsweise etwa 1,1 × 10⁻⁸ mol steigende Dosen Acetylcholin über einen Herzkatheter in die Koronararterien verabreicht, bis eine Wirkung eintritt oder die maximale Dosis von 1,1 x 10⁻⁶ mol erreicht wird.

Allerdings sind die für andere Anwendungen üblichen ACh-Dosierungen (z. B. in der Ophthalmologie) für diesen Test zu hoch, sodass Verdünnungen erforderlich sind. Diese müssen individuell und unmittelbar vor der Anwendung hergestellt werden, da ACh in wässriger Lösung instabil ist. Dies führt zu erheblichem Zeitaufwand, potenziellen Verdünnungsfehlern und einer eingeschränkten Verwendungsdauer der Lösungen aufgrund regulatorischer Vorgaben.

Einige Studien haben sich mit der Stabilität von ACh-Lösungen in unterschiedlichen Konzentrationen, sowohl hoch- als auch niedrigkonzentriert, befasst (Sletten et al., "Stability of acetylcholine chloride solution in autonomic testing", J Neurol Sci. 234:1-3 (2005)). Neue Untersuchungen zeigen zudem, dass niedrig konzentrierte ACh-Lösungen unter bestimmten Bedingungen stabil bleiben können (Neef et al., "Stability of Ready-To-Use-Solutions of Acetylcholine for Intracoronary Provocative Testing", Research Letter, JACC: Basic to Translational Science 10(1) (2025)), was für die praktische Anwendung relevant ist.

Ferner bietet ein in der DE 699 23 063 T2 beschriebenes pharmazeutisches Produkt zur ophthalmologischen Anwendung eine Lösung zur kurzfristigen Herstellung von ACh-Lösungen, ist jedoch für den intrakoronaren Einsatz nicht optimiert.

Die vorliegende Erfindung zielt darauf ab, die genannten Nachteile zu überwinden und die Implementierung des intrakoronaren ACh-Provokationstests in der klinischen Praxis durch eine verbesserte Methodik und Zusammensetzung zu erleichtern. Dies wird von der aktuellen Leitlinie der europäischen Gesellschaft für Kardiologie zum chronischen Koronarsyndrom auch propagiert (siehe auch Vrints et al. European Heart Journal, Volume 45, Issue 36, 21 September 2024, Seiten 3415-3537).

### Offenbarung der Erfindung

Die Aufgabe der vorliegenden Erfindung wird gelöst durch ein System zur Bereitstellung gebrauchsfertiger Acetylcholin-Injektionslösungen mit unterschiedlichen Acetylcholin-Konzentrationen, wobei das System aufweist: mindestens fünf erste Aufnahmeeinheiten, wobei jede der fünf ersten Aufnahmeeinheiten eine Menge (A; B; C; D; E) an Acetylcholin oder an einem Acetylcholin-Analogon als Feststoff enthält, und wobei die enthaltenen Mengen (A; B; C; D; E) an Acetylcholin oder an dem Acetylcholin-Analogon zwischen den fünf ersten Aufnahmeeinheiten unterschiedlich sind, derart, dass die Menge (A; B; C; D) an Acetylcholin oder an dem Acetylcholin-Analogon in vier der mindestens fünf ersten Aufnahmeeinheiten in jeder nachfolgenden ersten Aufnahmeeinheit größer ist als in der unmittelbar vorhergehenden ersten Aufnahmeeinheit, und die Menge (E) an Acetylcholin oder an dem Acetylcholin-Analogon in der fünften der mindestens fünften ersten Aufnahmeeinheit kleiner ist als die höchste Menge (D) in den vier ersten Aufnahmeeinheiten.

Die Aufgabe wird ferner gelöst durch die Verwendung des erfindungsgemäßen Systems in der Diagnose von koronaren vasomotorischen Störungen, bevorzugt Angina pectoris oder koronarer Herzkrankheit, insbesondere in einem intrakoronaren Acetylcholin-Provokationstest, wobei insbesondere die Mengen (A; B; C; D) an Acetylcholin oder an dem Acetylcholin-Analogon in den vier ersten Aufnahmeeinheiten für die linke Koronararterie vorgesehen sind, und die Menge (E) an Acetylcholin oder an dem Acetylcholin-Analogon in der fünften ersten Aufnahmeeinheit für die rechte Koronararterie vorgesehen ist.

Schließlich wird die Aufgabe durch ein Verfahren zum Herstellen gebrauchsfertiger Acetylcholin-Injektionslösungen mit unterschiedlichen Acetylcholin-Konzentrationen gelöst, wobei das erfindungsgemäße System eingesetzt wird.

Das erfindungsgemäße System ermöglicht vorteilhafterweise die kontrollierte und reproduzierbare Herstellung von Acetylcholin-Injektionslösungen mit definierten Konzentrationen, wodurch Variabilitäten durch manuelle Verdünnungsprozesse eliminiert werden. Da die Mengen an Acetylcholin oder dessen Analogon in den ersten Aufnahmeeinheiten exakt vorgegeben und abgestuft sind, wird das Risiko von Verdünnungsfehlern, die bei manuellen Zubereitungsschritten auftreten können, erheblich minimiert. Ferner reduziert die direkte Verfügbarkeit vordefinierter Acetylcholin-Konzentrationen den Zeitaufwand für die Vorbereitung der Lösungen, wodurch klinische und diagnostische Verfahren effizienter durchgeführt werden können.

Auch wird durch die Bereitstellung von Acetylcholin in fester Form innerhalb der ersten Aufnahmeeinheiten die chemische Stabilität des Wirkstoffs optimiert, was die Haltbarkeit der einzelnen Komponenten im erfindungsgemäßen System verlängert und den Bedarf an häufigen Neuzubereitungen verringert. Da keine offenen Verdünnungsschritte erforderlich sind, wird das Risiko mikrobieller oder chemischer Kontaminationen während der Herstellung der Injektionslösung erheblich reduziert. Die abgestufte Menge an Acetylcholin oder dessen Analogon innerhalb der ersten Aufnahmeeinheiten ermöglicht eine flexible Anpassung der Konzentration an spezifische diagnostische oder therapeutische Anforderungen.

Das erfindungsgemäße System ist daher u.a. geeignet für die Integration in automatisierte Dosierungs- und Injektionssysteme, wodurch eine präzise und reproduzierbare Anwendung in klinischen oder experimentellen Umgebungen gewährleistet wird.

Ferner kann durch die standardisierte Zubereitung und Bereitstellung der Acetylcholin-Lösungen die Compliance mit regulatorischen Anforderungen in Bezug auf Arzneimittelherstellung und -verwendung verbessert werden.

Ein weiterer Vorteil besteht darin, dass mit dem erfindungsgemäßen System keine komplexen Verdünnungsschritte erforderlich sind. So wird das Risiko menschlicher Fehler reduziert, was insbesondere in zeitkritischen klinischen Anwendungen von Vorteil ist. Erfindungsgemäß weist das System zumindest fünf, vorzugsweise genau 5, 6, 7, 8, 9, 10 oder mehr, erste Aufnahmeeinheiten auf. Dabei ist mit "Aufnahmeeinheit" jedes Behältnis mit umfasst, das zur Aufnahme und/oder Speicherung von Acetylcholin oder Acetylcholin-Analoga als Feststoff ausgelegt ist. Die zumindest 5 Aufnahmeeinheiten dienen als zentrales Element zur Bereitstellung von Acetylcholin bzw. Acetylcholin-Analoga innerhalb des Systems und stellt sicher, dass die Substanz bedarfsgerecht und in definierten Mengen zur Verfügung steht. Dabei bedeutet "mindestens fünf", dass das System fünf oder mehr solcher Einheiten umfasst, um eine flexible Anpassung der bereitgestellten Acetylcholin-Mengen zu ermöglichen, abhängig von den jeweiligen Anwendungsanforderungen. Jede der fünf ersten Aufnahmeeinheiten enthält eine Menge an Acetylcholin oder an einem Acetylcholin-Analogon als Feststoff. Ferner wird vorliegend, wie auch im betreffenden Gebiet, unter einem "Acetylcholin-Analogon" eine chemische Verbindung verstanden, die strukturell oder funktionell dem Acetylcholin ähnelt und in der Lage ist, die gleiche oder eine vergleichbare physiologische Wirkung, insbesondere als Neurotransmitter, auszuüben. Die enthaltenen Mengen (A; B; C; D; E) an Acetylcholin oder an dem Acetylcholin-Analogon unterscheiden sich zwischen den fünf ersten Aufnahmeeinheiten, derart, dass die Menge (A; B; C; D) an Acetylcholin oder an dem Acetylcholin-Analogon in mindestens vier der mindestens fünf ersten Aufnahmeeinheiten in jeder nachfolgenden ersten Aufnahmeeinheit größer ist als in der unmittelbar vorhergehenden ersten Aufnahmeeinheit, und die Menge (E) an Acetylcholin oder an dem Acetylcholin-Analogon in der fünften der mindestens fünf ersten Aufnahmeeinheit kleiner ist als die höchste Menge (D) in den vier der fünf ersten Aufnahmeeinheiten. Ferner soll "die Menge an Acetylcholin oder an dem Acetylcholin-Analogon ist in jeder nachfolgenden ersten Aufnahmeeinheit größer als in der unmittelbar vorhergehenden ersten Aufnahmeeinheit" bedeuten, dass die Masse oder Konzentration des in einer nachfolgenden ersten Aufnahmeeinheit enthaltenen Acetylcholins oder Acetylcholin-Analogons um mindestens eine definierte Einheit, wie bspw. Mikrogramm oder Mol, über der Menge der unmittelbar vorhergehenden ersten Aufnahmeeinheit liegt, um eine präzise und skalierbare Dosierung zu gewährleisten.

In anderen Worten gilt bspw. dann, wenn fünf erste Aufnahmeeinheiten im erfindungsgemäßen System vorliegen, mit einer Menge A in der ersten der fünf ersten Aufnahmeeinheiten, eine Menge B in der zweiten der fünf ersten Aufnahmeeinheiten, eine Menge C in der dritten der fünf ersten Aufnahmeeinheiten, eine Menge D in der vierten der fünf ersten Aufnahmeeinheiten, und eine Menge E in der fünften der fünf ersten Aufnahmeeinheiten: A < B < C < D, und E < D.

Dabei sind, insbesondere bei einem intrakoronaren Acetylcholin-Provokationstest, vorzugsweise die Mengen A, B, C und D bei einem für die linke Koronararterie vorgesehen und die Menge E für die rechte Koronararterie.

Gemäß einer Ausführungsform weist das erfindungsgemäße System die oben beschriebenen mindestens 5 ersten Aufnahmeeinheiten mit den angegebenen Mengen (A; B; C; D; E) an Acetylcholin oder an einem Acetylcholin-Analogon als Feststoff auf, sowie ein einziges Gefäß, bspw. eine Ampulle, das eine Verdünnungslösung aufweist.

Diese Ausführungsform hat den Vorteil, dass aus dem Gefäß dann nacheinander jeweils ein Volumen der Verdünnungslösung entnommen und in die Aufnahmeeinheiten zur Erreichung der gewünschten Acetylcholin-Konzentrationen gegeben werden kann.

Erfindungsgemäß liegt das Acetylcholin oder das Acetylcholin-Analogon als Feststoff vor, vorzugsweise als ein Salz, bestehend aus dem Acetylcholin-Kation und dem Anion einer geeigneten organischen oder anorganischen Säure, bspw. Essigsäure, Ameisensäure, Weinsäure, Zitronensäure, Gluconsäure, Glutaminsäure, Kohlensäure, Phosphorsäure, Schwefelsäure, oder Halogenwasserstoffsäure, insbesondere als Acetylcholinchlorid/Acetylcholinchlorid-Analogon oder Acetylcholinbromid/Acetylcholinbromid-Analogon.

Acetylcholin ist (2-(Trimethylammonium)ethyl)acetat und als solches ein Kation. Ein Vorliegen des Acetylcholin als Feststoff, der ein Salz ist, führt vorteilhafterweise zu einer erhöhten Stabilität des Acetylcholins, da dieses in wässrigem Milieu zersetzt wird. Vorliegend ist besonders vorteilhaft, wenn das Acetylcholin als Chlorid vorliegt, da Chlorid natürlicherweise im menschlichen Körper auftritt. Auch ist Acetylcholin in Form von Salzen, insbesondere und beispielswiese als Acetylcholinchlorid, stabiler. Dieses Salz ist ein weißes, kristallines Pulver, das kommerziell erhältlich ist.

Die Stabilität des Acetylcholins ist insbesondere bei saurem oder basischem pH-Wert reduziert. Deshalb ist es vorteilhaft, wenn neben dem Acetylcholin auch pharmazeutisch akzeptable Träger- und/oder Zusatzstoffe in den Aufnahmeeinheiten vorgesehen sind, welche den pH-Wert regulieren bzw. stabilisieren. Auf diese Weise wird die Stabilität des Acetylcholins nach einem Lösen in Wasser oder einer wässrigen Lösung erhöht.

Bevorzugte Mengen bzw. Mengenbereiche an Acetylcholin oder an dem Acetylcholin-Analogon als Feststoff betragen für die mindestens fünf Aufnahmeeinheiten wie folgt: in der ersten von vier der mindestens fünf ersten Aufnahmeeinheiten ist eine Menge an Acetylcholin oder an einem Acetylcholin-Analogon als Feststoff enthalten, wobei die Menge so bemessen ist, dass bei vollständiger Auflösung in einem definierten Volumen einer Verdünnungslösung eine Acetylcholin-/Acetylcholin-Analogon-Konzentration von zwischen 0,01 bis 1,0 µg/ml, vorzugsweise von zwischen 0,05 bis 0,8 µg/ml, noch bevorzugter zwischen 0,1 und 0,5 µg/ml, und weiter bevorzugt 0,2 µg/ml in der gebrauchsfertigen Injektionslösung erreicht wird, einschließlich jeweils der konkret angegebenen Grenzwerte; in der auf die erste nachfolgenden zweiten der vier mindestens fünf ersten Aufnahmeeinheiten eine Acetylcholin-/Acetylcholin-Analogon-Konzentration von zwischen 1,1 bis 3,0 µg/ml, vorzugsweise von zwischen 1,3 bis 2,7 µg/ml, noch bevorzugter zwischen 1,5 und 2,5 µg/ml, und weiter bevorzugt 2,0 µg/ml, einschließlich jeweils der konkret angegebenen Grenzwerte; in der auf die zweite nachfolgenden dritten der vier mindestens fünf ersten Aufnahmeeinheiten eine Acetylcholin-/Acetylcholin-Analogon-Konzentration von zwischen 9,1 bis 19,0 µg/ml, vorzugsweise von zwischen 9,5 bis 15,0 µg/ml, noch bevorzugter zwischen 9,5 und 12,0 µg/ml, und weiter bevorzugt 10 µg/ml, einschließlich jeweils der konkret angegebenen Grenzwerte; und in der vierten von vier der mindestens fünf ersten Aufnahmeeinheiten eine Acetylcholin-/Acetylcholin-Analogon-Konzentration von zwischen 19,1 bis 30,0 µg/ml, vorzugsweise von zwischen 19,5 bis 25,0 µg/ml, noch bevorzugter zwischen 19,5 und 23,0 µg/ml, und weiter bevorzugt 20 µg/ml, einschließlich jeweils der konkret angegebenen Grenzwerte; und wobei in einer fünften der fünf ersten Aufnahmeeinheiten, deren Menge kleiner ist als die höchste Menge in den vier ersten Aufnahmeeinheiten der mindestens fünft ersten Aufnahmeeinheiten eine Acetylcholin-/Acetylcholin-Analogon-Konzentration von zwischen 3,1 bis 9,0 µg/ml, vorzugsweise von zwischen 5,0 bis 9,0 µg/ml, noch bevorzugter zwischen 6,0 und 8,5 µg/ml, und weiter bevorzugt 8,0 µg/ml, einschließlich jeweils der konkret angegebenen Grenzwerte vorliegt.

Beispielhaft können dann, wenn jeweils gebrauchsfertige 10 ml-Injektionslösungen bereitgestellt werden sollen, in den fünf ersten Aufnahmeeinheiten die folgenden Mengen an Acetylcholin oder an einem Acetylcholin-Analogon als Feststoff vorliegen: in der ersten von vier der mindestens fünf ersten Aufnahmeeinheiten eine Menge von zwischen 0,1 µg bis 10 µg, vorzugsweise zwischen 0,5 µg bis 8 µg, noch bevorzugter zwischen 1,0 µg und 5,0 µg, und weiter bevorzugt 2,0 µg, jeweils einschließlich der angegebenen Grenzwerte; in der zweiten von vier der mindestsens fünf ersten Aufnahmeeinheiten eine Menge von zwischen 11 µg bis 30 µg, vorzugsweise zwischen 13 µg bis 27 µg, noch bevorzugter zwischen 15 µg und 25 µg, und weiter bevorzugt 20 µg, jeweils einschließlich der angegebenen Grenzwerte; in der dritten von vier der mindestens fünf ersten Aufnahmeeinheiten eine Menge von zwischen 91 µg bis 190 µg, vorzugsweise zwischen 95 µg bis 150 µg, noch bevorzugter zwischen 95 µg und 120 µg, und weiter bevorzugt 100 µg, jeweils einschließlich der angegebenen Grenzwerte; und in der vierten von vier der mindestens fünf ersten Aufnahmeeinheiten eine Menge von zwischen 191 µg bis 300 µg, vorzugsweise zwischen 195 µg bis 250 µg, noch bevorzugter zwischen 195 µg und 230 µg, und weiter bevorzugt 200 µg, jeweils einschließlich der angegebenen Grenzwerte; und wobei in der fünften der mindestens fünf ersten Aufnahmeeinheiten, deren Menge kleiner ist als die höchste Menge der vier von fünf ersten Aufnahmeeinheiten, eine Menge von zwischen 31 µg bis 90 µg, vorzugsweise zwischen 50 µg bis 90 µg, noch bevorzugter zwischen 60 µg und 85 µg, und weiter bevorzugt 80 µg, jeweils einschließlich der angegebenen Grenzwerte vorliegt.

Es versteht sich, dass die o.g. Mengen beispielhaft sind und auf die jeweiligen Anforderungen, den Patienten und die spezifische Verwendung individuell angepasst werden können, wobei wesentlicher Kern ist, dass die Mengen in den fünf ersten Aufnahmeeinheiten ansteigend sind.

Gemäß einer bevorzugten Ausführungsform weist das System ferner mindestens fünf zweite Aufnahmeeinheiten auf, wobei jede der fünf zweiten Aufnahmeeinheiten eine Menge an einer Verdünnungslösung enthält, und wobei jeweils eine erste Aufnahmeeinheit einer zweiten Aufnahmeeinheit zugeordnet ist.

Diese Ausführungsform hat den Vorteil, dass aufgrund der Zuordnung jeder der ersten Aufnahmeeinheiten mit Acetylcholin-Feststoff direkt einer zweiten Aufnahmeeinheit mit einer definierten Menge an Verdünnungslösung die Herstellung einer gebrauchsfertigen Injektionslösung erleichtert wird, da die exakte Verdünnung damit "automatisch" sichergestellt ist. Darüber hinaus können sich durch die Kombination von verschiedenen Mengen an Acetylcholin-Feststoff mit abgestuften Mengen an Verdünnungslösung unterschiedliche Konzentrationen bedarfsgerecht und schnell herstellen lassen, ohne dass zusätzliche manuelle Berechnungen oder Arbeitsschritte notwendig sind. Dadurch, dass jede Acetylcholin-Einheit bereits ihrer passenden Verdünnung zugeordnet ist, wird das Risiko von Dosierungsfehlern minimiert. Dies erhöht die Sicherheit, was insbesondere bei kritischen Anwendungen wie Injektionen kritisch ist. Ferner ermöglicht die direkte Zuordnung eine sofortige Zubereitung der Injektionslösungen, was Zeit spart und den gesamten Prozess effizienter gestaltet.

Vorteilhafterweise kann das System auch einfach um zusätzliche Aufnahmeeinheiten erweitert werden, um eine noch größere Bandbreite an Konzentrationen bereitzustellen, ohne die grundlegende Struktur zu ändern.

Die Verdünnungslösung ist dabei steril; vorzugsweise ist sie ausgewählt aus einem der folgenden: Kochsalzlösung (bspw. 0,45 % oder 0,9 % (w/v) Natriumchlorid, NaCl), Glukoselösung (bspw. 5 % oder 10 % (w/v) Dextrose), Wasser für Injektionszwecke (Aqua ad iniectabilia), Ringer-Lösung, Ringer-Laktat-Lösung (Hartmann-Lösung), Natriumhydrogencarbonat-Lösung.

Generelle, weitere Inhaltsstoffe der Verdünnungslösung können sein Zitronensäure, Salze der Zitronensäure, Calcium-Salze, Kaliumsalze, Glycin oder weitere Aminosäuren und weitere zur Herstellung von Injektionslösungen geeignete Bestandteile.

Gemäß einer bevorzugten Ausführungsform des erfindungsgemäßen Systems erfolgt die Zuordnung jeder der mindestens fünf ersten Aufnahmeeinheiten zu einer der mindestens fünf zweiten Aufnahmeeinheiten durch ein Kennzeichnungssystem, vorzugsweise bestehend aus Farben, Zahlen oder Symbolen. Jede erste Aufnahmeeinheit weist dabei eine Kennzeichnung auf, die mit der Kennzeichnung einer entsprechenden zweiten Aufnahmeeinheit übereinstimmt, sodass eine eindeutige Paarbildung gewährleistet ist. Insgesamt sind bei fünf ersten und fünf zweiten Aufnahmeeinheiten fünf unterschiedliche Kennzeichnungen vorgesehen, um die jeweils zugehörigen Aufnahmeeinheiten klar voneinander zu unterscheiden und eine fehlerfreie Zuordnung zu ermöglichen.

Gemäß einer weiteren Ausführungsform des erfindungsgemäßen Systems ist jeweils eine erste Aufnahmeeinheit und eine zweite Aufnahmeeinheit in einem gemeinsamen Behältnis verbunden zur Bildung von mindestens fünf gemeinsamen Behältnissen, mit zwischen jeweils einer ersten Aufnahmeeinheit und einer zweiten Aufnahmeeinheit vorgesehenen, zumindest teilweise aufhebbaren Barrieremitteln.

Durch die Verbindung einer ersten und einer zweiten Aufnahmeeinheit in einem gemeinsamen Behältnis wird der Transport, die Lagerung und die Bereitstellung deutlich erleichtert, da weniger Einzelteile gehandhabt werden müssen. Die vorgesehenen, zumindest teilweise aufhebbaren Barrieremittel ermöglichen darüber hinaus eine gezielte Freisetzung der Verdünnungslösung zur Vermischung mit dem Acetylcholin-Feststoff. Dies stellt sicher, dass die Lösung erst unmittelbar vor der Anwendung hergestellt wird, was die Stabilität erhöht. Mit dem erfindungsgemäßen System kann der Aufwand für manuelle Mischvorgänge weiter reduziert und eine schnelle, reproduzierbare Herstellung der Injektionslösungen ermöglicht werden. Auch bei dieser Ausführungsform können Verwechslungen ausgeschlossen und eine fehlerfreie Anwendung unterstützt werden, da jedes gemeinsame Behältnis genau ein Paar aus erster und zweiter Aufnahmeeinheit enthält.

In einer weiteren bevorzugten Ausführungsform des erfindungsgemäßen Systems ist das gemeinsame Behältnis eine Spritze oder eine Ampulle, mit zwischen den mindestens fünf ersten Aufnahmeeinheiten und den mindestens fünf zweiten Aufnahmeeinheiten vorgesehenen, zumindest teilweise aufhebbaren Barrieremitteln.

Dabei wird vorliegend unter einer "Ampulle" ein verschlossenes Behältnis, typischerweise aus Glas oder Kunststoff, verstanden, das eine Flüssigkeit oder einen Feststoff in steriler Umgebung enthält. Sie dient der Lagerung, dem Transport und der späteren Entnahme des Inhalts, wobei sie durch Brechen, Aufschneiden oder durch eine spezielle Öffnungsvorrichtung geöffnet wird. Unter einer "Spritze" wird ein medizinisches Instrument verstanden, bestehend aus einem zylindrischen Hohlkörper (Zylinder) mit einem beweglichen Kolben, das zur Aufnahme, Dosierung und Injektion von Flüssigkeiten dient. Sie ist in der Regel mit einer Nadel oder einem Anschluss versehen, um Flüssigkeiten präzise zu entnehmen oder zu applizieren.

Die Verwendung einer Spritze oder Ampulle als gemeinsames Behältnis bietet den Vorteil, dass auf erprobte, leicht handhabbare Lösungen zurückgegriffen werden kann. Dabei ermöglichen die mindestens teilweise aufhebbaren Barrieremittel eine unkomplizierte Freisetzung der Verdünnungslösung in den Bereich mit dem Acetylcholin-Feststoff, was eine sofortige Herstellung der gebrauchsfertigen Injektionslösung innerhalb eines einzigen Behältnisses ermöglicht.

Ferner wird das das Risiko von Materialverlusten, die beim Umfüllen entstehen könnten, minimiert, da die Verdünnung und die Applikation im selben Behältnis erfolgen. Die Herstellung der Lösung direkt in einer sterilen Spritze oder Ampulle reduziert außerdem das Risiko einer Kontamination erheblich, da keine zusätzlichen Transferprozesse notwendig sind. Schließlich verkürzt die sofortige Verfügbarkeit der gebrauchsfertigen Injektionslösung in einem Applikationsbehältnis die Vorbereitungszeit erheblich, was insbesondere in Notfallsituationen entscheidend sein kann.

Vorliegend wird unter "aufhebbare Barrieremittel" jede Vorrichtung oder Struktur verstanden, die das Vermischen oder den Kontakt zwischen zwei oder mehr Komponenten innerhalb eines Behältnisses zumindest teilweise verhindert, bis eine gezielte Aufhebung dieser Barriere erfolgt. Barrieremittel können mechanischer oder physikalischer Natur sein und sind so ausgelegt, dass sie durch manuelle, mechanische oder automatische Betätigung entfernt, durchbrochen oder umgangen werden können, um die Vermischung der getrennten Komponenten zu ermöglichen.

Gemäß bevorzugten Ausführungsformen des erfindungsgemäßen Systems können die Barrieremittel ausgewählt sein aus Membranversiegelungen, also dünne Membranen, die durch Druck, Drehung oder einen Kolbenhub durchstoßen werden können, um die Vermischung der Verdünnungslösung und des Acetylcholins zu ermöglichen; frangible, d.h. bruchfähige Verbindungen, insbesondere fragile Trennwände oder Sollbruchstellen, die durch mechanische Belastung, bspw. durch Knicken oder Drücken, gezielt geöffnet werden können; Septen mit Durchstichvorrichtung, insbesondere elastische Gummisepten, die mit einer Nadel durchstochen werden; schraub- oder Drehmechanismen, insbesondere drehbare Verschlüsse oder Kolben, die beim Verdrehen bspw. einen Kanal zwischen den beiden Aufnahmeeinheiten öffnen; Schiebedichtungen oder Kolbenventile, insbesondere bewegliche Dichtungen, die durch einen Kolben oder Schieber geöffnet werden können, um den Inhalt zweier Aufnahmeeinheiten zu vermischen; Foliensiegel mit Abziehmechanismus, also Folien, die durch Abziehen oder Einreißen entfernt werden können; Ventilsysteme, bspw. Einwegventile oder Rückschlagventile, die bei Betätigung einen Flüssigkeitsfluss zwischen den Aufnahmeeinheiten zulassen, aber eine vorzeitige Vermischung verhindern.

Gemäß einer weiteren bevorzugten Ausführungsform des erfindungsgemäßen Systems sind die mindestens fünf gemeinsamen Behältnisse strukturell gekoppelt, mit separaten ersten Aufnahmeeinheiten und zweiten Aufnahmeeinheiten.

Vorteilhafterweise ermöglicht die strukturelle Kopplung der mindestens fünf gemeinsamen Behältnisse eine platzsparende Anordnung, was Lagerung, Transport und Handhabung erleichtert. Auch können durch die Kopplung mehrere Einheiten gleichzeitig vorbereitet, transportiert oder appliziert werden, ohne dass einzelne Behältnisse separat entnommen oder verwaltet werden müssen. Dadurch sind auch alle notwendigen Konzentrationen in einem strukturell verbundenen System verfügbar, wodurch ein schneller Wechsel zwischen den gewünschten Konzentrationen möglich ist. Auch gewährleistet die strukturelle Kopplung eine einheitliche Lagerung und schützt die einzelnen Aufnahmeeinheiten vor physischen Einwirkungen, die zu Beschädigungen oder Kontamination führen könnten. Trotz der Kopplung bleiben die ersten und zweiten Aufnahmeeinheiten separat, sodass bei Bedarf einzelne Einheiten ausgetauscht oder ergänzt werden können, ohne das gesamte System zu ersetzen. Die strukturierte Anordnung der Behältnisse erleichtert eine systematische Kennzeichnung und Zuordnung, was Fehler bei der Auswahl der gewünschten Konzentration verhindert.

Gemäß einer bevorzugten Ausführungsform des erfindungsgemäßen Systems sind die Barrieremittel zwischen den mindestens fünf ersten Aufnahmeeinheiten und den mindestens fünf zweiten Aufnahmeeinheiten gleichzeitig oder unabhängig voneinander aufhebbar. Durch die Möglichkeit, die Barrieremittel unabhängig voneinander aufzuheben, kann der Nutzer gezielt eine einzelne Injektionslösung herstellen, ohne die anderen Aufnahmeeinheiten zu beeinflussen.

Auf der anderen Seite ermöglicht es die gleichzeitige Aufhebbarkeit aller Barrieremittel, mehrere gebrauchsfertige Lösungen gleichzeitig bereitzustellen, was in zeitkritischen Situationen, z.B. im medizinischen Bereich, besonders vorteilhaft sein kann.

Die Anwender können je nach Bedarf entweder eine einzelne Lösung zubereiten oder mehrere Lösungen parallel, ohne zusätzlichen Aufwand oder Wartezeiten.

In einer weiteren bevorzugten Ausführungsform des erfindungsgemäßen Systems, das neben den fünf ersten Aufnahmeeinheiten mindestens fünf zweite Aufnahmeeinheiten aufweist, und jede der fünf zweiten Aufnahmeeinheiten eine Menge an einer Verdünnungslösung enthält, und wobei jeweils eine erste Aufnahmeeinheit einer zweiten Aufnahmeeinheit zugeordnet ist, sind die fünf ersten Aufnahmeeinheiten von den mindestens fünf zweiten Aufnahmeeinheiten strukturell voneinander getrennt.

Diese Ausführungsform bietet den Vorteil, den Anwender flexibel entscheiden zu lassen, welche Mengen oder Konzentrationen gebraucht werden, da jede Einheit individuell zugänglich ist. Auch kann das System durch Hinzufügen oder Entfernen einzelner Aufnahmeeinheiten leicht erweitert oder angepasst werden, ohne die Integrität der übrigen Einheiten zu beeinträchtigen.

Bei dieser Ausführungsform können bspw. die zweiten Aufnahmeeinheiten bspw. über die weiter oben geschilderten Kennzeichnungssysteme, bspw. Farben, Zahlen, Symbole oder sonstige Markierungen, einer jeweiligen ersten Aufnahmeeinheit zugeordnet werden.

In bevorzugten Ausführungsformen sind die mindestens fünf ersten Aufnahmeeinheiten jeweils Ampullen, bzw. die fünf ersten Aufnahmeeinheiten sind in Ampullen enthalten, und die mindestens fünf zweiten Aufnahmeeinheiten jeweils Spritzen, bzw. die fünf zweiten Aufnahmeeinheiten sind in Spritzen enthalten, oder die mindestens fünf ersten Aufnahmeeinheiten sind jeweils Spritzen, oder darin enthalten, und die mindestens fünf zweiten Aufnahmeeinheiten sind jeweils Ampullen, oder darin enthalten.

Durch die wählbare Kombination - entweder Spritzen als erste Aufnahmeeinheiten und Ampullen als zweite oder umgekehrt - kann das System an verschiedene Anforderungen und Vorlieben der Anwender angepasst werden.

Spritzen als erste Aufnahmeeinheiten ermöglichen eine sofortige und präzise Dosierung des Acetylcholin-Feststoffs, während Ampullen als zweite Aufnahmeeinheiten eine sterile und sichere Bereitstellung der Verdünnungslösung gewährleisten - oder umgekehrt, je nach bevorzugter Handhabung. Darüber hinaus kann die Verwendung standardisierter Spritzen und Ampullen die Bedienung im klinischen Alltag erleichtern. Die Kombination aus Spritze und Ampulle - in beiden Varianten - ermöglicht eine rasche und präzise Herstellung der gebrauchsfertigen Injektionslösung durch einfache mechanische Vorgänge (z.B. Aufziehen oder Injizieren der Verdünnungslösung). Auch kann dieses System je nach Anforderung skaliert werden, da sowohl Ampullen als auch Spritzen in unterschiedlichen Volumina und Konfigurationen verfügbar sind.

Gemäß einer weiteren Ausführungsform des erfindungsgemäßen Systems sind den mindestens fünf ersten Aufnahmeeinheiten über ein automatisches oder halbautomatisches Zugabesystem jeweils Verdünnungslösungen zuführbar.

Ein automatisches oder halbautomatisches Zugabesystem gewährleistet eine exakte Dosierung der Verdünnungslösung, wodurch menschliche Fehler bei der Zubereitung minimiert werden. Die automatisierte Zuführung kann ferner den manuellen Aufwand erheblich reduzieren und ermöglicht eine schnelle Bereitstellung gebrauchsfertiger Injektionslösungen, insbesondere bei der gleichzeitigen Zubereitung mehrerer Einheiten.

Die standardisierte Zugabe sorgt dafür, dass jede Injektionslösung in identischer Konzentration und Qualität hergestellt wird, was die Reproduzierbarkeit und Zuverlässigkeit des Systems erhöht. Auch kann das Risiko von Kontaminationen durch manuelle Handhabung erheblich verringert werden. Ferner können durch programmierbare oder einstellbare Parameter im Zugabesystem unterschiedliche Mengen an Verdünnungslösung zugeführt werden, um verschiedene Konzentrationen flexibel und ohne zusätzlichen Aufwand herzustellen.

Das erfindungsgemäße System kann ferner Anwendungsinformationen aufweisen, die entweder auf jeder der fünf ersten und fünf zweiten Aufnahmeeinheiten, oder einem gemeinsamen Behältnis für jeweils eine erste und eine zweite Aufnahmeeinheit, wie weiter oben beschrieben, vorgesehen sind, und/oder einem Beipackmaterial des Systems aufgebracht und/oder elektronisch, bevorzugt über das Internet, zur Verfügung gestellt sein.

Wenn die Anwendungsinformationen auf jedem einzelnen Gefäß aufgebracht sind, kann das Gefäß aus Glas oder Plastik sein und mit Anwendungsinformationen bedruckt sein.

Wenn die Anwendungsinformationen auf einem Beipackmaterial des Systems aufgebracht sind, kann das Beipackmaterial Papier sein, welches mit Anwendungsinformationen bedruckt ist.

Wenn die Anwendungsinformationen elektronisch zur Verfügung gestellt werden, können die Anwendungsinformationen in einem öffentlich oder nur nach Identifikation zugänglichen, ggf. personalisierten, Bereich des Internets bereitgestellt sein.

Wie weiter oben erwähnt, betrifft die vorliegende Erfindung auch die Verwendung des oben beschriebenen erfindungsgemäßen Systems in allen seinen Ausführungsformen in der Diagnose von koronaren vasomotorischen Störungen, bevorzugt Angina pectoris oder koronarer Herzkrankheit, insbesondere in einem intrakoronaren Acetylcholin-Provokationstest, insbesondere in einem intrakoronaren Acetylcholin-Provokationstest zur Erkennung einer CVD während einer Herzkatheteruntersuchung, wobei insbesondere die Mengen (A; B; C; D) an Acetylcholin oder an dem Acetylcholin-Analogon in vier der mindestens fünf ersten Aufnahmeeinheiten für die linke Koronararterie vorgesehen sind, und die Menge (E) an Acetylcholin oder an dem Acetylcholin-Analogon in der fünften erste Aufnahmeeinheit für die rechte Koronararterie vorgesehen ist.

Vorteilhafterweise, und wie eingangs erwähnt, ermöglicht das erfindungsgemäße System die genaue und standardisierte Bereitstellung von Acetylcholin-Injektionslösungen, was für zuverlässige und wiederholbare Ergebnisse im intrakoronaren Acetylcholin-Provokationstest entscheidend ist. Durch die Bereitstellung unterschiedlicher Konzentrationen kann das System individuell an den Patienten angepasst werden, um verschiedene Reaktionen der Koronararterien zu testen und eine differenzierte Diagnostik zu ermöglichen. Das System gewährleistet darüber hinaus eine schnelle Zubereitung gebrauchsfertiger Lösungen direkt im klinischen Umfeld, was insbesondere bei zeitkritischen diagnostischen Verfahren wie dem Provokationstest vorteilhaft ist. Die standardisierte Bereitstellung und die optionalen automatisierten Zugabesysteme reduzieren das Risiko menschlicher Fehler und gewährleisten eine hohe Sterilität während des gesamten Verfahrens.

Die einfache Handhabung und die schnelle Verfügbarkeit verschiedener Konzentrationen sparen Zeit und Ressourcen im klinischen Alltag und erleichtern den Ablauf diagnostischer Prozeduren. Neben dem intrakoronaren Acetylcholin-Provokationstest kann das System auch in anderen diagnostischen und therapeutischen Anwendungen eingesetzt werden, was seine Vielseitigkeit unterstreicht.

Insbesondere kann das erfindungsgemäße System und der damit verbundene Acetylcholin-Test auch durch Bypass-Gefäße erfolgen, also insbesondere auch bei Patienten, die zuvor - also vor dem Acetylcholin-Test - einer koronaren Bypassoperation (Coronary artery bypass graft surgery (CABG)) unterzogen wurden, wobei hierbei die oben beschriebenen Dosierungen bzw. Mengen (A; B; C; D) (an Acetylcholin oder an dem Acetylcholin-Analogon) in den vier ersten Aufnahmeeinheiten eingesetzt werden, die für die linke Koronararterie vorgesehen sind.

Wie ferner bereits oben erwähnt betrifft die vorliegende Erfindung auch ein Verfahren zum Herstellen gebrauchsfertiger Acetylcholin-Injektionslösungen mit unterschiedlichen Acetylcholin-Konzentrationen, wobei das Verfahren die Schritte aufweist:
a) Bereitstellen eines erfindungsgemäßen Systems in einer der oben beschriebenen Ausführungsformen; und
b) Zugeben jeweils einer definierten Menge einer Verdünnungslösung zu jeder der mindestens fünf ersten Aufnahmeeinheiten.

Insbesondere ist hierbei bevorzugt, wenn Schritt b) automatisiert oder halbautomatisiert erfolgt.

Ferner betrifft die vorliegende Erfindung auch ein Verfahren zum Herstellen gebrauchsfertiger Acetylcholin-Injektionslösungen mit unterschiedlichen Acetylcholin-Konzentrationen, wobei das Verfahren die Schritte aufweist:
a) Bereitstellen eines erfindungsgemäßen Systems; und
b) Zugeben jeweils einer definierten Menge einer Verdünnungslösung zu jeder der mindestens fünf ersten Aufnahmeeinheiten aus mindestens fünf zweiten Aufnahmeeinheiten, die den mindestens fünf ersten Aufnahmeeinheiten jeweils zugeordnet sind.

Bei dieser Ausführungsform des erfindungsgemäßen Verfahrens kann bspw. das oben beschriebene Ampulle-Spritzen-Kombinationssystem eingesetzt werden, womit sich die für dieses System beschriebenen Vorteile auch für das Verfahren, in dem dieses eingesetzt wird, abbilden.

Schließlich betrifft die vorliegende Erfindung auch ein Verfahren zum Herstellen gebrauchsfertiger Acetylcholin-Injektionslösungen mit unterschiedlichen Acetylcholin-Konzentrationen, wobei das Verfahren die Schritte aufweist:
a) Bereitstellen eines erfindungsgemäßen Systems in einer der oben beschriebenen Ausführungsformen;
b) zumindest teilweises Aufheben der Barrieremittel, die jeweils zwischen einer ersten Aufnahmeeinheit und einer zweiten Aufnahmeeinheit vorgesehen sind; und
c) Zugeben jeweils einer definierten Menge einer Verdünnungslösung zu jeder der mindestens fünf ersten Aufnahmeeinheiten aus mindestens fünf zweiten Aufnahmeeinheiten, die den mindestens fünf ersten Aufnahmeeinheiten jeweils zugeordnet sind.

Bei den oben beschriebenen Verfahren werden als jeweils letzter Schritt durch das Zugeben der Verdünnungslösungen die in den ersten Aufnahmeeinheiten vorliegenden Acetylcholin-Mengen jeweils in Lösung gebracht, wodurch die gebrauchsfertigen Acetylcholin-Injektionslösungen hergestellt werden.

Bei dieser Ausführungsform des erfindungsgemäßen Verfahrens wird eines der oben beschriebenen Ausführungsformen des erfindungsgemäßen Systems eingesetzt, bei denen die fünf ersten und die fünf zweiten Aufnahmeeinheiten durch Barrieremittel voneinander getrennt sind. Die für die jeweiligen Ausführungsformen für diese Systeme beschriebenen Vorteile ergeben sich damit auch für das Verfahren, in dem sie eingesetzt werden.

Weitere Vorteile, Merkmale und Einzelheiten der vorliegenden Erfindung ergeben sich aus den beigefügten Figuren und der nachfolgenden Beschreibung. Es versteht sich, dass die dargestellten und beschriebenen Ausführungsformen lediglich beispielhaft sind und die Erfindung nicht darauf beschränkt ist.

### Kurze Beschreibung der Zeichnungen

**Fig. 1** zeigt eine schematische, nicht-maßstabsgetreue Darstellung einer ersten beispielhaften Ausführungsform des erfindungsgemäßen Systems, mit 5 Reihen von je zwei Aufnahmeeinheiten, also erste Aufnahmeeinheiten und zugeordnete zweite Aufnahmeeinheiten, die durch zumindest teilweise aufhebbare Barrieremittel voneinander getrennt sind);
**Fig. 2** zeigt eine schematische, nicht-maßstabsgetreue Darstellung einer zweiten beispielhaften Ausführungsform des erfindungsgemäßen Systems, mit 5 Spritzen und 5 zugeordneten Ampullen;
**Fig. 3** zeigt eine schematische, nicht-maßstabsgetreue Darstellung einer dritten beispielhaften Ausführungsform des erfindungsgemäßen Systems, mit 5 Spritzen, die jeweils zwei Aufnahmeeinheiten aufweisen, die durch zumindest teilweise aufhebbare Barrieremittel voneinander getrennt sind;
**Fig. 4** zeigt eine schematische, nicht-maßstabsgetreue Darstellung einer vierten beispielhaften Ausführungsform des erfindungsgemäßen Systems, bei dem den ersten Aufnahmeeinheiten automatisch bzw. halbautomatisch Verdünnungslösungen zugeführt werden.

### Ausführungsformen der Erfindung

### Detaillierte Beschreibung der Figuren

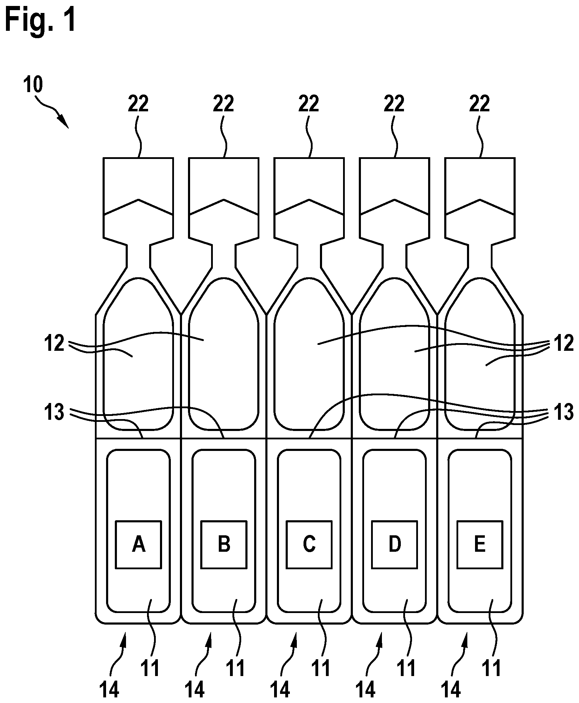

In Fig. 1 ist eine schematische, nicht-maßstabsgetreue Darstellung einer ersten beispielhaften Ausführungsform des erfindungsgemäßen Systems 10 gezeigt. Das hier beispielhaft gezeigte System umfasst 5 Reihen mit je einer ersten Aufnahmeeinheit 11 und einer zugeordneten zweiten Aufnahmeeinheit 12. Die jeweils zugeordneten Aufnahmeeinheiten 11 und 12 sind jeweils durch zumindest teilweise aufhebbare Barrieremittel 13 voneinander getrennt. Die Barrieremittel in dem in Fig. 1 gezeigten System 10 stellen bspw. eine Sollbruchstelle zwischen jeweils der ersten und zweiten Aufnahmeeinheit 11, 12 dar.

Jeweils eine erste Aufnahmeeinheit 11 und eine zweite Aufnahmeeinheit 12 sind in einem gemeinsamen Behältnis 14, hier eine Ampulle 22, verbunden. Beispielhaft, sowie in Fig. 1 gezeigt, sind die Behältnisse 14 strukturell gekoppelt, mit separaten ersten Aufnahmeeinheiten 11 und zweiten Aufnahmeeinheiten 12, die jeweils über aufhebbare Barrieremittel 13 voneinander getrennt sind.

In den ersten Aufnahmeeinheiten 11 sind unterschiedliche Mengen A, B, C, D, E an Acetylcholin als Feststoff enthalten, wobei die Acetylcholin-Menge B, die sich in der Aufnahmeeinheit 11 befindet, die auf die Aufnahmeeinheit 11 folgt, die die Acetylcholin-Menge A enthält, größer ist als die Menge A; ferner ist die Acetylcholin-Menge C, die sich in der Aufnahmeeinheit 11 befindet, die auf die Aufnahmeeinheit 11 folgt, die die Acetylcholin-Menge B enthält, größer ist als die Menge B; ferner ist die Acetylcholin-Menge D, die sich in der Aufnahmeeinheit 11 befindet, die auf die Aufnahmeeinheit 11 folgt, die die Acetylcholin-Menge C enthält, größer ist als die Menge C; und schließlich ist die Acetylcholin-Menge E, die sich in der Aufnahmeeinheit 11 befindet, die auf die Aufnahmeeinheit 11 folgt, die die Acetylcholin-Menge D enthält, kleiner als die Menge D. Anders gesprochen gilt damit A < B < C < D und E < D, was bedeutet, dass die Acetylcholin-Menge in vier von mindestens 5 ersten Aufnahmeeinheiten in jeder nachfolgenden Aufnahmeeinheit 11 größer ist als in der unmittelbar vorhergehenden Aufnahmeeinheit 11.

Die fünf zweiten Aufnahmeeinheiten 12 enthalten Mengen an Verdünnungslösungen a, b, c, d, und e, die in dem in Fig. 1 gezeigten Beispiel identisch sind. Bei Entfernen der Barrieremittel 13 können sich die in den fünft zweiten Aufnahmeeinheiten 12 vorgesehenen Verdünnungslösungen a, b, c, d, und e mit den jeweiligen Acetylcholin-Mengen A, B, C, D, E, die in den jeweils zugeordneten Aufnahmeeinheiten 11 vorliegen, vermischen, so dass das jeweils vorliegende Acetylcholin in Lösung geht, und die gebrauchsfertigen Acetylcholin-Injektionslösungen bereitgestellt werden können. Diese können bspw. jeweils mit einer Spritze (nicht gezeigt) aufgezogen und für den erwünschten Zweck, insbesondere für einen intrakoronaren Acetylcholin-Provokationstest, eingesetzt werden.

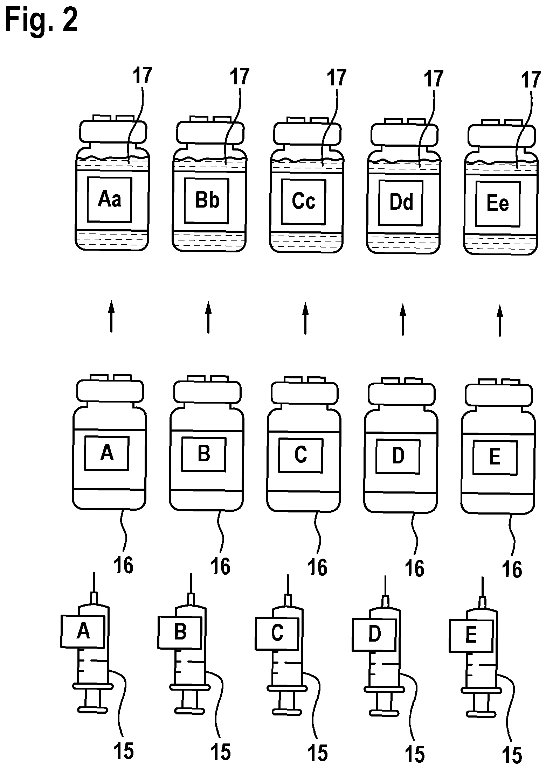

Fig. 2 zeigt eine schematische, nicht-maßstabsgetreue Darstellung einer zweiten beispielhaften Ausführungsform des erfindungsgemäßen Systems 10. Das in Fig. 2 gezeigte System umfasst beispielhaft fünf Spritzen 15, die im unteren Teil der Figur gezeigt sind, und fünf zugeordnete Ampullen 16, die in der Mitte der Figur gezeigt sind. Die fünf Ampullen 16 bilden bzw. enthalten jeweils eine der fünf ersten Aufnahmeeinheiten 11, in welchen die unterschiedlichen Mengen A, B, C, D, und E an Acetylcholin als Feststoff vorliegen, wobei auch hier gilt: A < B < C < D und E < D.

Die im unteren Teil der Figur 2 gezeigten Spritzen 15 bilden bzw. enthalten jeweils eine der fünf zweiten Aufnahmeeinheiten 12, in denen Verdünnungslösungen a, b, c, d, e vorliegen, deren Mengen in der hier beispielhaft gezeigten Ausführungsform identisch sind. In den Ampullen 16 sind die Aufnahmeeinheiten 11 vorgesehen, bzw. die Ampullen 16 bilden die Aufnahmeeinheiten 11. In diesen sind unterschiedliche Acetylcholin-Mengen A, B, C, D, E vorgesehen, wobei auch hier gilt: A < B < C < D und E < D. Die Ampullen weisen ein teilweises entfernbares Barrieremittel 13 in Form eines durchstechbaren Septums auf (nicht gezeigt). Jeweils eine Spritze 15 ist einer Ampulle 16 zugeordnet, und nach Durchstechen kann die Verdünnungslösung a, b, c, d, e mit den jeweils zugeordneten Acetylcholin-Mengen A, B, C, D, und E vermengt werden wodurch sich das Acetylcholin jeweils lösen kann und die fertigen Injektionslösungen 17 (auch mit Aa, Bb, Cc, Dd, Ee zur Verdeutlichung der unterschiedlichen Konzentrationen bezeichnet) bereitgestellt werden, die unterschiedliche Acetylcholin-Konzentrationen enthalten. Aus den Ampullen 16 können dann die fertigen Injektionslösungen 17 bspw. mittels einer Spritze (nicht gezeigt) entnommen und für den erwünschten Zweck, insbesondere für einen intrakoronaren Acetylcholin-Provokationstest, eingesetzt werden.

Es versteht sich, dass auch umgekehrt die unterschiedlichen Acetylcholin-Mengen in den Aufnahmeeinheiten 11 von Spritzen vorliegen können, und die Verdünnungslösungen in den Aufnahmeeinheiten 12 der Ampullen.

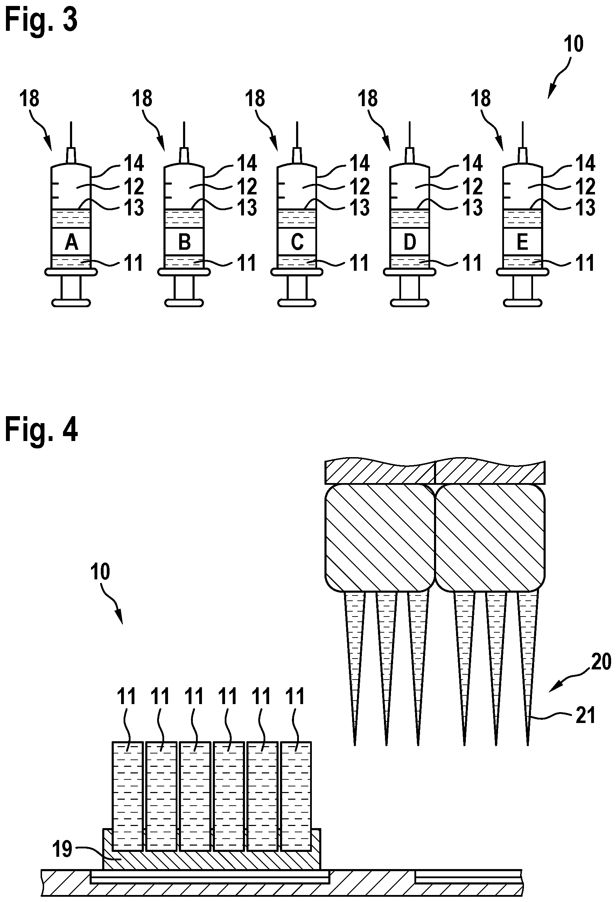

Fig. 3 zeigt eine weitere schematische, nicht-maßstabsgetreue Darstellung einer dritten beispielhaften Ausführungsform des erfindungsgemäßen Systems 10. Hier sind fünf Behältnisse 14 in Form von Spritzen 18 gezeigt, die jeweils eine erste Aufnahmeeinheit 11 und eine zugeordnete zweite Aufnahmeeinheit 12 aufweisen. Die jeweils zugeordneten Aufnahmeeinheiten 11 und 12 sind jeweils durch zumindest teilweise aufhebbare Barrieremittel 13 voneinander getrennt. Die Barrieremittel in dem in Fig. 3 gezeigten System 10 stellen bspw. Membranen oder Septen dar, die durchbrochen oder durchstochen werden können, um die Barriere aufzuheben.

Auch hier, analog zu dem in Fig. 1 gezeigten Ausführungsbeispiel, sind jeweils eine erste Aufnahmeeinheit 11 und eine zweite Aufnahmeeinheit 12 in einem gemeinsamen Behältnis 14, hier eine Spritze, verbunden. Anders als in der in Fig. 1 gezeigten Ausführungsform sind die Behältnisse 14 aber nicht strukturell gekoppelt, sondern voneinander getrennt.

In den ersten Aufnahmeeinheiten 11 sind unterschiedliche Mengen A, B, C, D, E an Acetylcholin (als Feststoff) enthalten, wobei auch hier - analog zu Fig. 1 - für die Acetylcholin-Mengen gilt: A < B < C < D und E < D, was bedeutet, dass die Acetylcholin-Menge in vier von mindestens fünf nachfolgenden Aufnahmeeinheiten 11 größer ist als in der unmittelbar vorhergehenden Aufnahmeeinheit 11.

Die fünf zweiten Aufnahmeeinheiten 12 enthalten Mengen an Verdünnungslösungen a, b, c, d, und e, die in dem in Fig. 3 gezeigten Beispiel identisch sind. Bei Entfernen der Barrieremittel 13 können sich die in den fünft zweiten Aufnahmeeinheiten 12 vorgesehenen Verdünnungslösungen a, b, c, d, und e mit den jeweiligen Acetylcholin-Mengen A, b, C, D, E, die in den jeweils zugeordneten Aufnahmeeinheiten 11 vorliegen, vermischen, so dass das jeweils vorliegende Acetylcholin in Lösung geht, und die gebrauchsfertigen Acetylcholin-Injektionslösungen bereitgestellt werden können. Diese können in der Spritze direkt für den erwünschten Zeck, insbesondere für einen intrakoronaren Acetylcholin-Provokationstest, eingesetzt werden.

Fig. 4 zeigt eine schematische, nicht-maßstabsgetreue Darstellung einer vierten beispielhaften Ausführungsform des erfindungsgemäßen Systems 10. Hier sind beispielhaft sechs erste Aufnahmeeinheiten 11 vorgesehen (wobei auch fünf oder mehr als sechs vorgesehen sein können), die bspw. Ampullen oder Vials sein können, die auf einem Rack 19 bereitgestellt werden. Der Rack 19 dient dabei als Halterung, in der mehrere Aufnahmeeinheiten in einer definierten Anordnung fixiert sind. Diese sechs ersten Aufnahmeeinheiten 11 weisen Acetylcholin als Feststoff in unterschiedlichen Mengen A, B, C, D, E, F auf.

Den Aufnahmeeinheiten 11 werden für die Herstellung der gebrauchsfertigen Acetylcholin-Injektionslösungen anschließend automatisiert, bspw. über eine Pipettiereinheit 20, Verdünnungslösungen zupipettiert. Dabei wird jede erste Aufnahmeeinheit 11 durch ein Identifikationsmerkmal (wie eine Farbe, Zahl oder ein Symbol, Code) gekennzeichnet, sodass ein automatisiertes System (nicht gezeigt) erkennt, welches Gefäß für welchen Verdünnungsschritt oder Pipettiervorgang vorgesehen ist. Eine automatisierte Pipettiereinheit bewegt dann Pipettiernadeln 21 präzise zu den jeweiligen Aufnahmeeinheiten 11. Die Nadel kann dann die Verdünnungslösung zuführen zur Herstellung einer gebrauchsfertigen Acetylcholin-Injektionslösung mit unterschiedlichen Acetylcholin-Konzentrationen.

Ein solches System 10 kann entweder vollständig automatisiert oder teilautomatisiert sein. In beiden Fällen stellt es sicher, dass die benötigte Menge an Verdünnungslösung in die entsprechenden Aufnahmeeinheiten 11 pipettiert wird, um die gewünschten Konzentrationen zu erreichen.

## Patentansprüche

1. System (10) zur Bereitstellung gebrauchsfertiger Acetylcholin-Injektionslösungen (17) mit unterschiedlichen Acetylcholin-Konzentrationen, wobei das System aufweist:
- mindestens fünf erste Aufnahmeeinheiten (11), wobei jede der fünf ersten Aufnahmeeinheiten eine Menge (A; B; C; D; E) an Acetylcholin oder an einem Acetylcholin-Analogon als Feststoff enthält, und wobei die enthaltenen Mengen (A; B; C; D; E) an Acetylcholin oder an dem Acetylcholin-Analogon zwischen den fünf ersten Aufnahmeeinheiten (11) unterschiedlich sind, derart, dass die Menge (A; B; C; D) an Acetylcholin oder an dem Acetylcholin-Analogon in vier der mindestens fünf ersten Aufnahmeeinheiten in jeder nachfolgenden ersten Aufnahmeeinheit größer ist als in der unmittelbar vorhergehenden ersten Aufnahmeeinheit (11), und die Menge (E) an Acetylcholin oder an dem Acetylcholin-Analogon in der fünften der mindestens fünf ersten Aufnahmeeinheit kleiner ist als die höchste Menge (D) in den vier ersten Aufnahmeeinheiten.

2. System (10) nach Anspruch 1, wobei das System ferner
- mindestens fünf zweite Aufnahmeeinheiten (12) aufweist, wobei jede der fünf zweiten Aufnahmeeinheit (12) eine Menge (a, b, c, d, e) an einer Verdünnungslösung enthält, und wobei jeweils eine erste Aufnahmeeinheit (11) einer zweiten Aufnahmeeinheit (12) zugeordnet ist.

3. System (10) nach Anspruch 2, wobei jeweils eine erste Aufnahmeeinheit (11) und eine zweite Aufnahmeeinheit (12) in einem gemeinsamen Behältnis (14) verbunden sind zur Bildung von mindestens fünf gemeinsamen Behältnissen, mit zwischen jeweils einer ersten Aufnahmeeinheit (11) und einer zweiten Aufnahmeeinheit (12) vorgesehenen, zumindest teilweise aufhebbaren Barrieremitteln (13).

4. System (10) nach Anspruch 3, wobei das gemeinsame Behältnis (14) eine Spritze (18) oder eine Ampulle (22) ist, mit zwischen den mindestens fünf ersten Aufnahmeeinheiten (11) und den mindestens fünf zweiten Aufnahmeeinheiten (12) vorgesehenen, zumindest teilweise aufhebbaren Barrieremitteln (13).

5. System (10) nach Anspruch 3 oder 4, wobei die mindestens fünf gemeinsamen Behältnisse (14) strukturell gekoppelt sind, mit separaten ersten Aufnahmeeinheiten (11) und zweiten Aufnahmeeinheiten (12).

6. System (10) nach einem der Ansprüche 3 bis 5, wobei die Barrieremittel (13) zwischen den mindestens fünf ersten Aufnahmeeinheiten (11) und den mindestens fünf zweiten Aufnahmeeinheiten (12) gleichzeitig oder unabhängig voneinander aufhebbar sind.

7. System (10) nach Anspruch 2, wobei die mindestens fünf ersten Aufnahmeeinheiten (11) von den mindestens fünf zweiten Aufnahmeeinheiten (12) strukturell voneinander getrennt sind.

8. System (10) nach Anspruch 7, wobei die mindestens fünf ersten Aufnahmeeinheiten (11) jeweils Ampullen (16) und die mindestens fünf zweiten Aufnahmeeinheiten (12) jeweils Spritzen (15) sind, oder wobei die mindestens fünf ersten Aufnahmeeinheiten (11) jeweils Spritzen (15) und die mindestens fünf zweiten Aufnahmeeinheiten jeweils Ampullen (16) sind.

9. System (10) nach Anspruch 1, wobei den mindestens fünf ersten Aufnahmeeinheiten (11) über ein automatisches oder halbautomatisches Zugabesystem jeweils Verdünnungslösungen (a, b, c, d, e) zuführbar sind.

10. Verwendung eines Systems (10) nach einem der vorhergehenden Ansprüche in der Diagnose von koronaren vasomotorischen Störungen, bevorzugt Angina pectoris oder koronarer Herzkrankheit, insbesondere in einem intrakoronaren Acetylcholin-Provokationstest, insbesondere wobei die Mengen (A; B; C; D) an Acetylcholin oder an dem Acetylcholin-Analogon in den vier ersten Aufnahmeeinheiten für die linke Koronararterie vorgesehen sind, und die Menge (E) an Acetylcholin oder an dem Acetylcholin-Analogon in der fünften ersten Aufnahmeeinheit für die rechte Koronararterie vorgesehen ist.

11. Verfahren zum Herstellen gebrauchsfertiger Acetylcholin-Injektionslösungen mit unterschiedlichen Acetylcholin-Konzentrationen, wobei das Verfahren die Schritte aufweist:
a) Bereitstellen eines Systems (10) nach einem der Ansprüche 1 bis 9; und
b) Zugeben jeweils einer definierten Menge einer Verdünnungslösung (a, b, c, d, e) zu jeder der mindestens fünf ersten Aufnahmeeinheiten (11).

12. Verfahren nach Anspruch 10, wobei Schritt b) automatisiert oder halbautomatisiert erfolgt.

13. Verfahren zum Herstellen gebrauchsfertiger Acetylcholin-Injektionslösungen mit unterschiedlichen Acetylcholin-Konzentrationen, wobei das Verfahren die Schritte aufweist:
a) Bereitstellen eines Systems (10) nach einem der Ansprüche 2 bis 9; und
b) Zugeben jeweils einer definierten Menge einer Verdünnungslösung (a, b, c, d, e) zu jeder der mindestens fünf ersten Aufnahmeeinheiten (11) aus mindestens fünf zweiten Aufnahmeeinheiten (12), die den mindestens fünf ersten Aufnahmeeinheiten (11) jeweils zugeordnet sind.

14. Verfahren zum Herstellen gebrauchsfertiger Acetylcholin-Injektionslösungen mit unterschiedlichen Acetylcholin-Konzentrationen, wobei das Verfahren die Schritte aufweist:
a) Bereitstellen eines Systems (10) nach einem der Ansprüche 3 bis 9;
b) zumindest teilweises Aufheben der Barrieremittel (13), die jeweils zwischen einer ersten Aufnahmeeinheit (11) und einer zweiten Aufnahmeeinheit (12) vorgesehen sind; und
c) Zugeben jeweils einer definierten Menge einer Verdünnungslösung (a, b, c, d, e) zu jeder der mindestens fünf ersten Aufnahmeeinheiten (11) aus mindestens fünf zweiten Aufnahmeeinheiten (12), die den mindestens fünf ersten Aufnahmeeinheiten (11) jeweils zugeordnet sind.
